# EUROPEAN PATENT APPLICATION

(11) **EP 4 792 827 A1**
(43) Date of publication of application: **19.08.2026**
(21) Application number: 23955315.9
(22) Date of filing: 04.12.2023
(51) Int. Cl.: A61K 38/19, A61P 17/00

(54) **METHOD FOR PREVENTING, REDUCING OR MITIGATING SENESCENCE-RELATED CHANGES, INJURIES OR SYMPTOMS OF SKIN**

(30) Priority: 13.10.2023 CN 202311328212
(71) Applicant: Renerval Biotherapeutics (Shanghai) Co., Ltd., Shanghai 201203 (CN)
(72) Inventor: WANG, Wenyuan, Shanghai 201203 (CN)
(74) Representative: Hoffmann Eitle
(86) International application number: PCT/CN2023/136268
(87) International publication number: WO 2025/076959

(57) **Abstract**

Provided are a method for preventing, reducing or improving senescence-related changes, injuries or signs of skin, and a use of a platelet factor 4 in preparation of a composition for use in the method.

## Description

### Technical Field

This invention relates to methods for preventing, reducing or improving senescence-related changes, injuries or signs of skin, and to compositions containing platelet factor 4 for use in such methods.

### Background Art

Senescence is defined as a gradual process of physiological decline that leads to physical weakness, age-related diseases, and ultimately death. Cellular senescence is closely related to physiological processes such as tumor suppression, tissue repair, embryogenesis, and body aging, and is a cellular state during these physiological processes (see Non-Patent Reference 1). Cellular senescence often occurs in response to endogenous or exogenous stress, and exhibits the following prominent features: 1) permanent cell cycle arrest; 2) senescence-associated secretory phenotype (*i.e.*, a collective term for pro-inflammatory cytokines and chemokines, growth regulators, angiogenic factors and matrix metalloproteinases (MMPs) secreted by senescent cells); 3) macromolecular damage (such as DNA damage); 4) metabolic disorders (such as impaired mitochondrial function and blocked ATP synthesis) (see Non-Patent Reference 2). Similar to other organs, skin is affected not only by intrinsic aging (such as chronological age) but also by environmental factors that accelerate aging.

Fibroblasts are the main component of connective tissue. Skin connective tissue is responsible for connecting all histologically different skin cells and tissues into a functional organ. Fibroblasts in the skin are capable of synthesizing and organizing extracellular matrix (ECM), and maintain close intercellular communication with neighboring cells and tissues. Therefore, cellular senescence of skin fibroblasts may directly or indirectly cause changes in skin homeostasis or skin aging. It has been reported that senescent fibroblasts are in a state of long-term proliferative arrest, and secrete pro-inflammatory senescence-associated secretory phenotypes (SASPs) into the surrounding environment, inducing local chronic inflammation, reducing the release of important growth factors, and accelerating the degradation of the extracellular matrix, thereby resulting in tissue degeneration and skin aging (see Non-Patent Reference 3).

Mature platelet factor 4 (PF4, also known as CXCL4) is a small polypeptide containing 70 amino acids synthesized by megakaryocytes. PF4 is generally stored in platelet α-granules and can form a non-covalently linked tetramer under physiological conditions (see Non-Patent Reference 4). The primary physiological function of PF4 is to promote blood coagulation. In addition, PF4 has been reported to participate in various physiological processes including inflammatory response and immune regulation. However, the relationship between platelet factor 4 and aging remains at a very early exploratory stage. Related studies are focused onPF4 as an inflammation-related factorwith potential to ameliorate cognitive function aging, see, e.g., Non-Patent References 5 to 7.

To date, it reamains unclear whether PF4 is involved in other aging-related physiological and/or pathological changes. Furthermore, there has been no report that PF4 can directly affect the activity of skin fibroblasts.

### References

Non-Patent Reference 1: Di Micco, R., et al., Cellular senescence in ageing: from mechanisms to therapeutic opportunities. Nat Rev Mol Cell Biol, 2021.22(2): p.75-95;
Non-Patent Reference 2: Gorgoulis, V., et al., Cellular Senescence: Defining a Path Forward. Cell, 2019, 179(4): p. 813-827;
Non-Patent Reference 3: Meinhard Wlaschek et al., Connective Tissue and Fibroblast Senescence in Skin Aging. J Invest Dermatol. 141(4S):985-992;
Non-Patent Reference 4: Kowalska, MA, L.Rauova, and M.Poncz, Role of the platelet chemokine platelet factor 4 (PF4) in hemostasis and thrombosis. Thromb Res, 2010.125 (4): p.292-6;
Non-Patent Reference 5: Schroer, AB, et al., Platelet factors attenuate inflammation and rescue cognition in ageing. Nature, 2023, 620(7976): p.1071-1079;
Non-Patent Reference 6: Park, C., et al., Platelet factors are induced by longevity factor klotho and enhance cognition in young and aging mice. Nat Aging, 2023.3 (9): p.1067-1078;
Non-Patent Reference 7: Leiter, O., et al., Platelet-derived exerkine CXCL4/platelet factor 4 rejuvenates hippocampal neurogenesis and restores cognitive function in aged mice. Nat Commun. 2023.14 (1): p.4375.

### Summary of the Invention

Through in-depth research, the inventors of the present application have discovered for the first time that platelet factor 4 can act directly on skin fibroblasts, effectively prevent and delay fibroblasts from entering a senescent state, and even revert senescent fibroblasts into a youthful state, thereby preventing, delaying skin aging or restoring the skin to a youthful state.

Therefore, one object of the present invention is to provide the use of platelet factor 4 in preparation of compositions for preventing, reducing or improving senescence-related changes, injuries or symptoms of skin.

Another object of the present invention is to provide a method for preventing, reducing or improving senescence-related changes, injuries or signs of skin, the method comprising administering to a subject a composition comprising an effective amount of platelet factor 4.

Another object of the present invention is to provide the use of platelet factor as the sole active ingredient in the preparation of compositions for repairing skin injuries.

Another object of the present invention is to provide a composition comprising an effective amount of platelet factor as the sole active ingredient for use and methods disclosed herein.

### The Description of Drawings

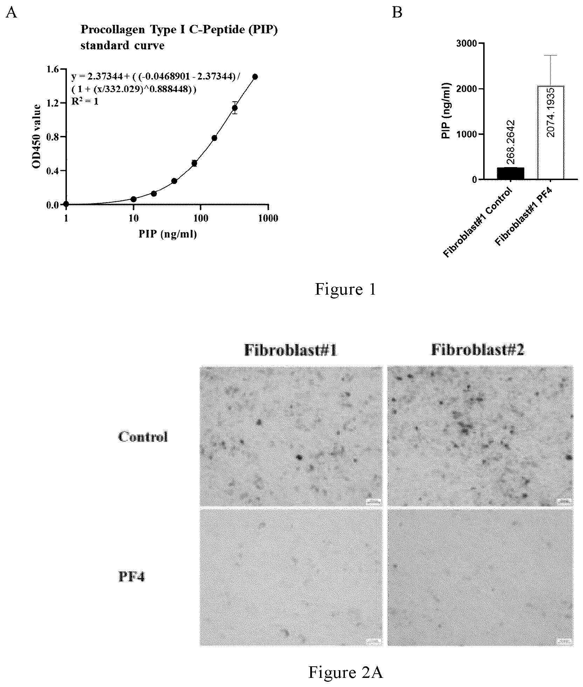

Figure 1 is a bar chart showing the enhancement of skin collagen release by platelet factor 4 in Example 1: Figure 1A shows the standard curve of collagen, and Figure 1B shows the differences in collagen secretion by cells under different culture conditions;

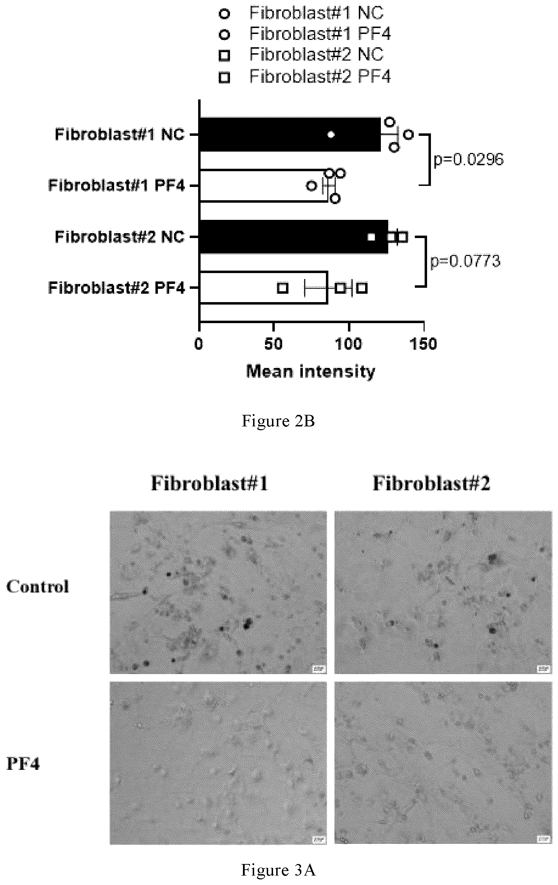

Figure 2 shows the β-galactosidase staining results of skin fibroblasts in Example 2. Figure 2A shows a representative staining photograph capturedby optical microscopy, with a scale bar of 20 µm. Figure 2B is a bar chart showing the statistical results of the gray values of staining analyzed by ImageJ for the control group (NC) and the experimental group (PF4).

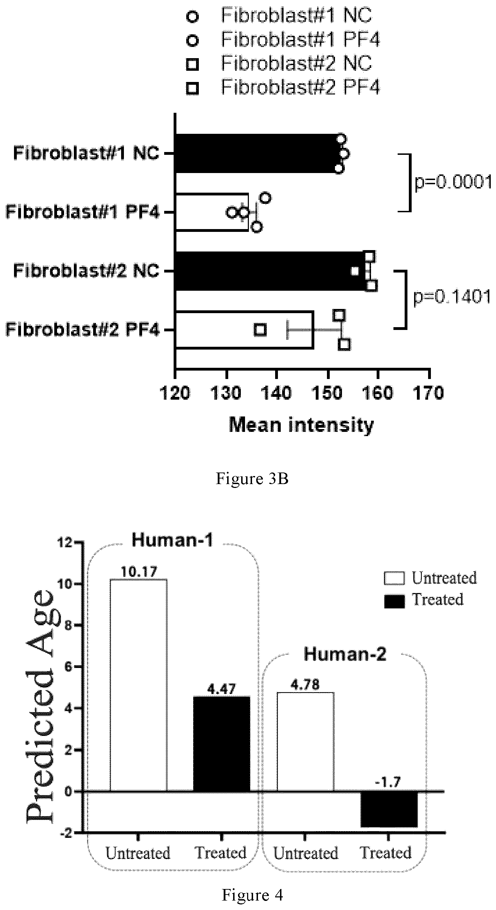

Figure 3 shows the β-galactosidase staining results of skin fibroblasts in Example 3. Figure 3A shows a representative staining photograph capturedby optical microscopy, with a scale bar of 20 µm. Figure 3B is a bar chart showing the statistical results of the gray values of staining analyzed by ImageJ for the control group (NC) and the experimental group (PF4).
Figure 4 shows the age of skin fibroblasts assessed by DNA methylation at senescence-related sites in Example 4.

### Detailed Description of the Preferred Embodiment

The present invention will be further described below with reference to specific embodiments. It should be understood that these embodiments are only used to illustrate the present invention and are not intended to limit the scope of protection of the present invention. Those skilled in the art can easily understand other advantages and effects of the present invention from the content disclosed in this specification. This invention can also be implemented or applied through other different specific embodiments, and various details in this specification can also be modified or changed based on different viewpoints and applications without departing from the spirit of this invention.

### I. Definition

Unless otherwise defined, all technical and scientific terms used in the present invention have the same meaning as commonly understood by one of ordinary skill in the art. It should be understood that the terminology used in the embodiments of the present invention is for describing specific particular implementations and not for limiting the scope of protection of this invention; in the specification and claims of the present invention, unless otherwise expressly stated in the text, the singular forms "a," "an," and "the" include the plural forms. When numerical ranges are given in the embodiments, it should be understood that, unless otherwise stated in the present invention, the two endpoints of each numerical range and any value between the two endpoints may be selected.

Unless explicitly stated or obvious from the context, the term "about" as used herein shall be understood to be within the normal tolerance range in the field, such as within 2 standard deviations of the mean. "About" can be understood as being within 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, or 0.01% of the stated value. Unless otherwise apparent from the context, all numerical values provided in this article are modified by the term "about".

As used herein, the term "senescence" refers to the process by which cells gradually decline in their functional activities, such as their ability to proliferate and differentiate and their physiological functions, over time during life activities. Senescent cells undergo morphological and metabolic changes, chromatin remodeling, changes in gene expression, cell cycle arrest, and secretion of senescence-associated secretory phenotypes (SASPs). During skin aging, a series of aging characteristics generally regarded as undesirable arise, which manifest as various changes, injuries, or signs in the structure and/or appearance of the skin, including but not limited to the appearance of fine lines and deep wrinkles, increased skin fragility, atrophy, decreased thickness and/or tautness, skin sagging, reduced firmness, fullness, flexibility and/or suppleness, deteriorated skin texture, reduced or lost barrier function, changes in facial contours, changes in radiance and/or brightness, reduced moisture, etc. Each of these characteristics increases with age, and therefore the term "senescence-related" has the same meaning as "age-related" herein. In the present application, the aging characteristics of skin are mainly caused by the decreased functional activity of dermal fibroblasts.

As used herein, the term "prevent" and its synonymous variations refer to delaying the appearance and occurrence of skin aging features to any degree. As used herein, the term "reduce" and its synonyms refer to lowering the appearance frequency or decreasing the severity of skin aging characteristics. As used herein, the term "improve" and its synonyms refer to reducing the number and/or decreasing the severity of existing skin aging characteristics. Non-limiting examples of preventing, reducing, or improving skin aging characteristics include, for example, maintaining and restoring skin elasticity; maintaining and restoring youthful skin status; improving procollagen and/or collagen production; regenerating collagen; increasing collagen production; delaying fibroblast aging; repairing fibroblast damage; preventing, reducing, or improving dermatological signs associated with chronological aging, photoaging, hormonal aging, and/or actinic aging; preventing, reducing, or improving skin cell injuries caused by ultraviolet radiation; preventing, reducing, or improving the appearance of fine lines and/or wrinkles; reducing or improving skin fragility; preventing, reducing, or improving the loss of glycosaminoglycans and/or collagen; preventing, reducing, or improving of skin atrophy; preventing, reducing, or improving the reduction in skin thickness and/or tautness; preventing, reducing, and/or improving of skin sagging; improving the firmness, fullness, flexibility and/or suppleness of the skin; improving skin texture and/or promoting restoration of skin texture to its original state; improving of skin barrier function; improving the appearance of skin contour; restoring skin radiance and/or brightness; supplementing skin constituents reduced by aging and/or menopause; increasing skin cell proliferation and/or replication; increasing skin cell metabolism reduced by aging and/or menopause; improving skin hydration; enhancing skin thickness; or any combination thereof. In some embodiments, preventing, reducing, or improving the appearance of fine lines and/or wrinkles may be selected from reducing the visibility of facial fine lines and wrinkles, facial wrinkles on the cheeks and forehead, vertical wrinkles between the eyes, horizontal wrinkles above the eyes and around the mouth, marionette lines, and in particular deep wrinkles or folds; preventing, reducing, and/or diminishing the appearance and/or depth of fine lines and/or wrinkles; improving the appearance of infraorbital fine lines and/or orbital fine lines; and reducing the appearance of crow's feet.

In some embodiments, the present disclosure also provides the use of platelet factor 4 as the sole active ingredient in the preparation of compositions for repairing skin injuries. In some embodiments, the skin injury is selected from acute or chronic wounds or scars, traumatic wounds caused by surgery, burns, radiation or accidents, and a tendency for spontaneous or induced contusions on the limbs.

As used herein, the term "platelet factor 4" broadly refers to natural PF4, its analogues, or its active fragments from any source (e.g., human or other non-human primates). The term emcompasses variants obtained by substituting or deleting one or more amino acid residues in native PF4, or by adding one or more amino acid residues to the sequence of native PF4, wherein the variants do not significantly alter the biological activity of the resulting product compared to native PF4. Variants of PF4 can be naturally occurring variants, such as splice variants or allelic variants, or they can be prepared using artificial synthesis and/or site-directed mutagenesis techniques or other known techniques suitable for this purpose. In some embodiments, the platelet factor 4 disclosed herein is human platelet factor 4. In a preferred embodiment, the platelet factor 4 disclosed herein has the amino acid sequence as shown in SEQ ID No:1 (EAEEDGDLQCLCVKTTSQVRPRHITSLEVIKAGPHCPTAQLIATLKNGRKICLDLQ APLYKKIIKKLLES):
In the present invention, the conserved amino acid substitutions are well known to those skilled in the art. The following six groups are examples of amino acids that are considered to be conserved substitutes for each other:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

In some embodiments, the platelet factor 4 disclosed herein may have an amino acid sequence with more than 75% identity to the amino acid sequence shown in SEQ ID No:1 and possess essentially the same biological activity as SEQ ID NO:1. For example, it may have an amino acid sequence identity of 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

Platelet factor 4 may exist in the composition in various states. It may be a single component, or in the form of a complex with other proteins or other substances to further stabilize its activity, such as a fusion protein. It may also exist separately from other components and be mixed prior to use. When present separately from other components, it may be in the form of a dry powder or an aqueous solution. In some embodiments, the platelet factor 4 content in the compositions disclosed herein is 2 ng/ml or higher. For example, the platelet factor 4 content may be 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181 182, 183, 184, 185, 186, 187, 188, 189, 190, 191, 192, 193, 194, 195, 196, 197, 198, 199, 200, 250, 300, 350, 400, 450, 500, 550, 600, 650, 700, 750, 800, 850, 900, 1000, 1100, 1200, 1300, 1400, 1500, 1600, 1700, 1800, 1900, 2000, 2500, 3000, 3500, 4000, 4500, or 5000 ng/ml. In a preferred embodiment, the platelet factor 4 content in the composition disclosed herein is 5 to 5000 ng/ml. In a more preferred embodiment, the platelet factor 4 content in the composition disclosed herein is 10 to 200 ng/ml.

In some embodiments of the present invention, the compositions disclosed herein further include pharmaceutically, cosmetically, dermatologically, or physiologically acceptable excipients. In a preferred embodiment, the excipient is a polymer.

In some embodiments of the present invention, the compositions disclosed herein are in the form of gels, tinctures, creams, ointments, lotions or aerosol sprays.

In some embodiments of the present invention, the compositions disclosed herein are selected from skin care products, cosmetics, pharmaceuticals, or additives of skin care products, cosmetics, and pharmaceuticals. In a preferred embodiment, the skincare product is selected from facial cleansers, toners, tone-up creams, facial masks, lotions, face creams, eye creams, essentces, makeup primer, makeup primer sprays, and sunscreens. The skincare products disclosed herein are formulated by mixing appropriate amounts of active platelet factor 4, moisturizers, tonics, thickeners, pearlescent agents, fragrances, and water. In a preferred embodiment, the cosmetic is selected from concealer pens, foundation creams, BB creams, pressed powders, loose powders, eyeshadow palettes, blushes, highlighters, and lip glosses. The cosmetics disclosed herein are prepared by formulating and compounding various raw materials (e.g., oily raw materials, powdery raw materials, gelling raw materials, surfactants, solvent raw materials, fragrances, dyes, pigments, etc.) with an appropriate amount of active platelet factor 4 added thereto. In a preferred embodiment, the pharmaceuticals disclosed herein are selected from sprays, creams, ointments, or gels. The administration methods of the pharmaceuticals may include wiping, spraying, wet dressing, coating, washing, and fumigation.

As used herein, "administration" includes topical application to a skin region of the subject. In a preferred embodiment, the skin region to which the product is administrated is a skin region prone to wrinkle formation or aging skin region. In a more preferred embodiment, the skin region to which the substance is administrated is the skin on the face, neck, hands, torso, and limbs of the subject, or any combination thereof. In some embodiments, the aging skin region to which the compositions disclosed herein are administrated is subjected to a conditionselected from: fine or coarse wrinkles, reduced skin thickness, and reduced elasticity and firmness.

As used herein, "subject" means human and non-human animal, provided that its skin structure contains fibroblasts. In some implementation schemes, subjects are selected from humans, horses, dogs, and cats. In a preferred embodiment, the subjects are humans.

As described above, the uses, methods, and compositions (e.g., reagents, skin care products, cosmetics, pharmaceuticals, etc.) disclosed herein have the following beneficial effects:

Based on the finding that platelet factor 4 can alleviate inflammation and improve aging-related cognitive function in mice, the present application discovers for the first time that exogenous platelet factor 4 protein can delay or even reduce senescence of skin fibroblasts, thereby promoting the release of collagen by skin fibroblasts, alleviating skin aging, enhancing skin repair function, maintaining and restoring skin elasticity and youthful skin status. The discovery of novel functions of platelet factor 4 according to the present application fills a gap in the field of skin anti-aging, and has enormous application prospects in the medical and cosmetic industries.

### Experimental objectives and materials

Unless otherwise stated, the experimental methods, detection methods and preparation methods disclosed in this invention all adopt conventional techniques in this technical field and related fields. In addition to the specific methods, equipment, and materials used in the embodiments, based on the knowledge of those skilled in the art and the description of this invention, any prior art methods, equipment, and materials similar to or equivalent to those described, equipment, and materials in the embodiments of this invention can be used to implement this invention.

Platelet factor 4 can be obtained commercially, for example from Peprotech, Cat. No. 300-16, or from Sigma-Aldrich, catalog number SRP3142.

The use of skin fibroblasts #1 and #2 in the present application was approved by the Ethics Committee of Huashan Hospital Affiliated to Fudan University. Skin fibroblast #1 was derived from the skin of a 67-year-old woman, and skin fibroblast #2 was derived from the skin of a 40-year-old man.

The culture medium for skin fibroblasts is: DMEM + 10% FBS + 1% GlutaMax + 10 ng/ml bFGF, wherein the components are as follows:
DMEM: Gbico, Cat. No. 11965-092,
GlutaMax (100X): Gbico, Cat. No. 35050-061,
FBS: Sigma, Cat. No. F8318-500ML,
b-FGF: Peprotech, Cat. No. 100-18B.

### Example 1:

The example describes the effect of platelet factor 4 on the ability of skin fibroblasts to produce collagen.

A collagen standard curve was determined using the Procollagen Type I C-Peptide (PIP) EIA kit (purchased from TAKARA, Cat. No. MK101) according to the manufacturer's instructions. A Procollagen Type I C-Peptide (hereinafter referred to as PIP) solution with a concentration of 640 ng/ml was prepared and serially diluted twofold to obtain diluted solutions of 320, 160, 80, 40, 20 and 10 ng/ml. Ultrapure water ddH₂O without PIP was used as a blank control. The serially diluted solutions were incubated with anti-PIP antibody at 37°C for 3 hours. Then, washing was performed using washing buffer (DPBS, SH30028.02). Substrate TMBZ (3,3',5,5'-tetramethylbenzidine) solution was added, and the reaction was allowed to proceed at room temperature for 15 minutes, followed by the addition of stop solution to terminate the reaction. Absorbance at OD450nm was measured using a microplate reader (Thermo Scientific, model 1530). Using Prism software, a best-fit curve was determined as the standard curve, with the absorbance value as the vertical axis and the PIP concentration (ng/ml) as the horizontal axis. The results are shown in Figure 1A.

Sskin fibroblasts #1 were seeded into a 24-well plate at 0.8 × 10⁵ cells/well and cultured in the above-mentioned skin fibroblast culture medium with 1 ml per well. Immediately after seeding, platelet factor 4 (PF4) was added to the culture medium of the experimental group (n=3) to a final concentration of 100 ng/ml; an equal amount of solvent water was added to the control group (n=3). After incubation for 3 days in an incubator at 37°C and 5% CO₂, the supernatant was collected and subjected to ELISA measurement as described above. The concentration of procollagen type I C peptide in the cell culture medium of each well in the control and experimental groups was determined according to the standard curve. The results are shown in Figure 1B.

Figure 1B shows that, compared with the control group without the addition of platelet factor 4, collagen secreted by cells in the experimental group increased by 7.74 fold, reaching 2074 ng/ml. This indicates that platelet factor 4 can significantly enhance the ability of skin fibroblasts to synthesize and secrete collagen.

### Example 2:

The example describes the effect of platelet factor 4 on the senescence state of skin fibroblasts.

Skin fibroblasts #1 and #2 were seeded into 24-well plates at 0.8 × 10⁵ cells/well, 1 ml of skin fibroblast culture medium was added to each well. Platelet factor 4 (PF4) was added to the culture medium of the experimental group (n=2) to a final concentration of 100 ng/ml; an equal amount of solvent water was added to the control group (n=2). Cells were incubated for 3 days in an incubator at 37°C and 5% CO₂. Then, β-galactosidase staining was performed in each well using the kit (purchased from Beyotime, Cat. No. C0602) according to the manufacturer's instructions. Specifically, after removing the culture medium, cells were washed once with PBS or HBSS, and then were added with 1 ml of fixative solution to fix at room temperature for 15 minutes. After removing the fixative solution, cells were washed thoroughly with PBS or HBSS, and were added with 1 ml of staining working solution to each well. After incubation at 37°C overnight, the staining results were observed and counted under a conventional optical microscope. The final staining results are shown in Figure 2.

In Figure 2A, blue staining indicates the presence of active β-galactosidase in cells, which is a marker enzyme that increases during the aging process of cells. A large number of blue-strained cells were observed in the two control group cells. In contrast, in the experimental group cells supplemented with platelet factor 4, the number of cells capable of being stained blue was significantly reduced, and the blue color intensity was also significantly lighter. In Figure 2B, statistical analysis of the staining gray values in random visual fields of the control and experimental groups using ImageJ software revealed that the reduction in galactosidase staining exhibited statistical significance in both cell lines. This indicates that platelet factor 4 can significantly inhibit or delay the entry of skin fibroblasts into a senescence state and reduce the senescence degree of fibroblasts.

### Example 3:

The example describes the effect of platelet factor 4 on skin fibroblasts that have already shown injuries by senescence. The example uses an ultraviolet irradiation model (see, e.g., Florence Debacq-Chainiaux et al., UV, stress and aging. Dermato-Endocrinology 4:3,236-240; Alessio De Magis et al., UV-induced G4 DNA structures recruit ZRF1 which prevents UV-induced senescence. Nat Commun. 2023 Oct 23; 14(1):6705; M Helenius et al., Attenuation of NF-kappaB signaling response to UVB light during cellular senescence. Exp Cell Res. 1999 Apr 10; 248(1):194-202) as an exemplary model of senescent skin fibroblasts. Those skilled in the art will know that the results will be equally applicable to other senescence-related injury models.

Skin fibroblasts #1 and #2 were seeded in 24-well plates at 0.1 x 10⁵ cells/well, and 1 ml of skin fibroblast culture medium was added to each well.The cells were irradiated simultaneously with a 30W UV lamp at 22°C for 15 minutes. After irradiation, platelet factor 4 (PF4) was added to the culture medium of the experimental group (n=2) to a final concentration of 200 ng/ml; an equal amount of solvent water was added to the control group (n=2). The cells were incubated for 1 day in an incubator at 37°C and 5% CO₂. Then, the senescence state of skin fibroblasts was detected by the same β-galactosidase staining method as in Example 2, and the results are shown in Figure 3.

In Figure 3A, almost no blue staining was observed in skin fibroblasts cultured in the presence of platelet factor 4, as compared with the control group. In Figure 3B, statistical analysis of the staining gray values in random visual fields using ImageJ software showed that the reduction in galactosidase staining was statistically significant both cell lines. This indicates that platelet factor 4 is also capable of repairing senescence injuries that has already occurred in skin fibroblasts caused by external or internal pro-aging factors.

### Example 4:

The example describes the effect of platelet factor 4 on the epigenetics and cellular age of skin fibroblasts.

Skin fibroblasts #1 and #2 were seeded in 24-well plates at 0.1 x 10⁵ cells/well, and 1 ml of skin fibroblast culture medium was added to each well. Platelet factor 4 (PF4) was added to the culture medium of the experimental group (n=2) to a final concentration of 200ng/ml; an equal amount of solvent water was added to the control group (n=2). Cells were incubated for 3 days in an incubator at 37°C and 5% CO₂. Then, the culture medium was discarded, and the digested fibroblasts were collected after trypsin treament. Total DNA was extracted using a kit (QIAGEN, catalog number: 69504) according to the manufacturer's instructions. The age of the cultured cells was then evaluated using known methods (see, example,g,, Non-Patent Reference 8: Atef Zaguia, Deepak Pandey et al., DNA Methylation Biomarkers-Based Human Age Prediction Using Machine Learning. Computational Intelligence and Neuroscience, Vol. 2022, Article ID 8393498). Specifically, the extracted genomic DNA was subjected to bisulfite conversion, and the DNA methylation status of 77 age-related loci (including cg22736354, cg19283806, cg18473521, cg02228185, cg06493994, cg19761273, cg01820374, and cg09809672, etc.) was determined. Based on the methylation status of these loci, the cellular age was then determined using the machine learning and AI algorithms described in Non-Patent Reference 8. The results are shown in Figure 4.

In Figure 4, compared with the control skin fibroblasts, the cellular age of cells cultured in the presence of platelet factor 4 was significantly decreased, indicating that platelet factor 4 can effectively reverse the senescenceof skin fibroblasts and restore them to a more youthful state.

The above description is only preferred embodiments of the present invention and is not a limitation of the present invention in any form or substance. It should be noted that those skilled in the art can make several improvements and additions without departing from the method of the present invention, and these improvements and additions should also be considered within the scope of protection of the present invention. Any modifications, alterations, and variations made by those skilled in the art without departing from the spirit and scope of this invention, based on the disclosed technical content, shall be equivalent embodiments of this invention. Furthermore, any modifications, alterations, and variations made to the above embodiments based on the essential technology of this invention shall still fall within the scope of this invention's technical solution.

## Claims

1. Use of platelet factor 4 in preparation of compositions for preventing, reducing or improving senescence-related changes, injuries or signs of skin.

2. The use according to claim 1, wherein the senescence-related changes, injuries or signs of skin are caused by decline in functional activities of skin fibroblasts.

3. The use according to any one of the preceding claims, wherein the preventing, reducing or improving of senescence-related changes, injuries or signs of skin is selected from a group consisting of:
maintaining and restoring skin elasticity; maintaining and restoring youthful skin status; improving procollagen and/or collagen production; regenerating collagen; increasing collagen production; delaying fibroblast aging; repairing fibroblast damage; preventing, reducing, or improving dermatological signs associated with chronological aging, photoaging, hormonal aging, and/or actinic aging; preventing, reducing, or improving skin cell injuries caused by ultraviolet radiation; preventing, reducing, or improving the appearance of fine lines and/or wrinkles; reducing or improving skin fragility; preventing, reducing, or improving the loss of glycosaminoglycans and/or collagen; preventing, reducing, or improving of skin atrophy; preventing, reducing, or improving the reduction in skin thickness and/or tautness; preventing, reducing, and/or improving of skin sagging; improving the firmness, fullness, flexibility and/or suppleness of the skin; improving skin texture and/or promoting restoration of skin texture to its original state; improving of skin barrier function; improving the appearance of skin contour; restoring skin radiance and/or brightness; supplementing skin constituents reduced by aging and/or menopause; increasing skin cell proliferation and/or replication; increasing skin cell metabolism reduced by aging and/or menopause; improving skin hydration; enhancing skin thickness; or any combination thereof.

4. The use according to claim 3, wherein the preventing, reducing, or improving the appearance of fine lines and/or wrinkles is selected from reducing the visibility of facial fine lines and wrinkles, facial wrinkles on the cheeks and forehead, vertical wrinkles between the eyes, horizontal wrinkles above the eyes and around the mouth, marionette lines, and in particular deep wrinkles or folds; preventing, reducing, and/or diminishing the appearance and/or depth of fine lines and/or wrinkles; improving the appearance of infraorbital fine lines and/or orbital fine lines; and reducing the appearance of crow's feet.

5. The use according to any one of the preceding claims, wherein the composition further comprises a pharmaceutically, cosmetically, dermatologically, or physiologically acceptable excipient, wherein the excipient is preferably a polymer.

6. The use according to any one of the preceding claims, wherein the composition is in the form of a gel, tincture, cream, ointment, lotion, or aerosol spray.

7. The use according to any one of the preceding claims, wherein the composition is selected from skin care products, cosmetics, pharmaceuticals, or additives to skin care products, cosmetics, or pharmaceuticals;
preferably, the skincare product is selected from facial cleansers, toners, tone-up cream, facial masks, lotions, face creams, eye creams, essences, makeup primers, makeup primer sprays, or sunscreens;
preferably, the cosmetic is selected from concealer pens, foundation creams, BB creams, pressed powders, loose powders, eyeshadow palettes, blushes, highlighters, or lip glosses;
preferably, the pharmaceutical is selected from sprays, ointments, creams, or gels.

8. The use according to any one of the preceding claims, wherein the composition comprises platelet factor 4 in an effective amount of 2 ng/ml or more, preferably 5 to 5000 ng/ml, and more preferably 10 to 200 ng/ml.

9. The use according to any one of the preceding claims, wherein the administration comprises topical administration to a skin region of a subject, preferably a skin region prone to wrinkle formation or aging skin region, more preferably skin on the face, neck, hands, torso, and limbs of the subject, or any combination thereof.

10. The use according to any one of the preceding claims, wherein the aging skin region is subjected to a condition selected from the group consisting of fine or coarse wrinkles, reduced skin thickness, reduced elasticity and firmness.

11. The use according to any one of the preceding claims, wherein the platelet factor 4 is a human platelet factor 4, preferably having the amino acid sequence as set forth in SEQ ID NO:1.

12. The use according to any one of the preceding claims, wherein the subject is a mammal selected from a human, horse, dog, and cat.

13. A method for preventing, reducing or improving senescence-related changes, injuries or signs of skin, comprising administering to a subject a composition comprising an effective amount of platelet factor 4.
